# EUROPEAN PATENT SPECIFICATION

(11) **EP 3 379 488 B1**
(45) Date of publication and mention of the grant of the patent: **24.02.2021**
(21) Application number: 17202309.5
(22) Date of filing: 17.11.2017
(51) Int. Cl.: G06T 5/00

(54) **MEDICAL IMAGE DISPLAYING APPARATUS AND MEDICAL IMAGE DISPLAYING METHOD**
MEDIZINISCHE BILDANZEIGEVORRICHTUNG UND MEDIZINISCHES BILDANZEIGEVERFAHREN
APPAREIL ET PROCÉDÉ D'AFFICHAGE D'IMAGE MÉDICALE

(30) Priority: 24.03.2017 KR 20170037669
(43) Date of publication of application: 26.09.2018
(73) Proprietor: Samsung Medison Co., Ltd., Hongcheon-gun, Gangwon-do, 25108 (KR)
(72) Inventor: Lee, Jun-kyo, Gangwon-do (KR); Kang, Tae-wook, Seoul (KR); Choi, Dong-il, Seoul (KR); Lee, Min-woo, Seoul (KR)
(74) Representative: Hylarides, Paul Jacques

(56) References cited:
- EP-A1- 2 339 534
- WO-A1-2010/113633
- US-A1- 2005 238 218
- US-A1- 2010 322 490
- US-B1- 7 605 821

## Description

### BACKGROUND

### 1. Field

The present disclosure relates to a medical image displaying method and a medical image displaying apparatus.

### 2. Description of the Related Art

Medical image systems provide an image of a live body and are being used in various fields. Examples of medical image systems include a magnetic resonance image (MRI) system, a computed tomography (CT) system, a positron emission tomography (PET)-CT system, and an ultrasound system.

For effective diagnosis of diseases and treatment of patients, medical image systems need to accurately and efficiently provide medical image information. Accordingly, medical image displaying apparatuses capable of effectively transmitting object information included in a medical image are in demand.

WO2010/113633 describes techniques for displaying a composed image made up of a current ultrasound image and a previously stored 3D tomographic image. The current ultrasound image includes an unclear shadow region where the luminance is higher or lower than usual. The unclear shadow region is replaced in the ultrasound image with the image of the corresponding region of a slice of the stored 3D tomographic image, the slice corresponding to the current ultrasound image.

EP2339534 relates to reduction of specular reflections in an image, wherein a specular reflection region in a target image is detected and replaced, at least partially, by an image area from a reference image for use i.a. in medical imaging e.g. in endoscopy. A first target image is analysed to detect a specular reflection region and an area surrounding it, analysing a second reference image to detect therein an area that matches the surrounding area previously detected in the first target image, whereby the area of the second reference image surrounded by this matching area corresponds to the specular reflection region of the first target image and is used to at least partially replace the specular reflection region in the first image.

### SUMMARY

Provided are medical image displaying apparatuses capable of predicting a region of an object distorted or lost by a shadow or an artifact in a real-time medical image, by using a feature point of the object.

Provided are medical image displaying apparatuses capable of predicting the region of the object distorted or lost in the real-time medical image, and reconstructing the region of the object, based on a result of the prediction.

Provided are medical image displaying apparatuses capable of reconstructing and displaying a reference image of the object, based on image information of the reconstructed region of the object in the real-time medical image.

Provided are computer-readable recording media having recorded thereon a computer program for executing a method of operating the medical image displaying apparatus.

According to an aspect of an embodiment, a medical image displaying method includes displaying a medical image of an object captured in real time, and a first reference image of the object registered with the medical image; obtaining image information of a region adjacent to a first region within the medical image, wherein the first region is a distorted region of the medical image with respect to a shape of the object; predicting image information of the first region, based on the image information of the region adjacent to the first region; and reconstructing a region corresponding to the object within the first reference image, based on the predicted image information of the first region, and displaying the reconstructed region.

The obtaining of the image information of the region adjacent to the first region includes at least one of: extracting a feature point of the object from the region adjacent to the first region; extracting a brightness value of the region adjacent to the first region; extracting a contour of the region adjacent to the first region; extracting variations in pixel values of the region adjacent to the first region by using an image processing filter; extracting histogram information of the region adjacent to the first region; extracting one or more differences between the pixel values of the region adjacent to the first region; and extracting shape information of the region adjacent to the first region.

The obtaining of the image information of the region adjacent to the first region includes: determining, as the first region, a region having low image quality within the region corresponding to the object in the medical image; and obtaining the image information of the region adjacent to the first region.

The predicting of the image information of the first region includes: determining a shape of the region adjacent to the first region, based on the image information of the region adjacent to the first region; and predicting a shape of the first region based on the determined shape of the region adjacent to the first region, wherein the predicted shape of the first region mirrors the determined shape of the region adjacent to the first region with respect to an arbitrary axis.

The predicting of the image information of the first region includes: determining a shape of the region adjacent to the first region, based on the image information of the region adjacent to the first region; and predicting a shape of the first region according to a predetermined pattern, when the determined shape of the region adjacent to the first region corresponds to the predetermined pattern.

The reconstructing of the region corresponding to the object and displaying the reconstructed region within the first reference image includes: reconstructing the region corresponding to the object within the first reference image, based on the predicted image information of the first region; and displaying the reconstructed region within the first reference image such that the reconstructed region is distinguished from an original region in the first reference image.

The medical image displaying method further includes reconstructing the first region within the medical image and displaying the reconstructed first region such that the reconstructed first region overlaps the medical image.

When a type of the first reference image is different from a type of the medical image, the reconstructing of the region corresponding to the object and displaying the reconstructed region within the first reference image includes: reconstructing the first region within the medical image; converting coordinate information of the reconstructed first region from a coordinate system of the medical image to a coordinate system of the first reference image; and displaying the region corresponding to the object within the first reference image, based on the converted coordinate information of the reconstructed first region.

The medical image displaying method further includes: displaying the obtained image information of the region adjacent to the first region and the predicted image information of the first region; correcting the predicted image information of the first region, based on a user input; and reconstructing the region corresponding to the object and displaying the reconstructed region within the first reference image, based on the corrected image information of the first region.

The medical image displaying method further includes magnifying and displaying the region corresponding to the object within the medical image, wherein the reconstructing of the region corresponding to the object and displaying the reconstructed region within the first reference image, based on the predicted image information of the first region, comprises magnifying and displaying the reconstructed region within the first reference image.

According to an aspect of another embodiment, a medical image displaying apparatus includes: a display configured to display a medical image of an object captured in real time, and a first reference image of the object registered with the medical image; and a processor configured to obtain image information of a region adjacent to a first region within the medical image, predict image information of the first region, based on the image information of the region adjacent to the first region, and reconstruct a region corresponding to the object within the first reference image, based on the predicted image information of the first region, wherein the first region is a distorted region of the medical image with respect to a shape of the object, wherein the display displays the reconstructed region..

The processor obtains the image information of the region adjacent to the first region by performing at least one of: extracting a feature point of the object from the region adjacent to the first region; extracting a brightness value of the region adjacent to the first region; extracting a contour of the region adjacent to the first region; extracting variations in pixel values of the region adjacent to the first region by using an image processing filter; extracting histogram information of the region adjacent to the first region; extracting one or more differences between the pixel values of the region adjacent to the first region; and extracting shape information of the region adjacent to the first region.

The processor determines, as the first region, a region having a low image quality within the region corresponding to the object in the medical image.

The processor determines a shape of the region adjacent to the first region, based on the image information of the region adjacent to the first region, and predicts a shape of the first region based on the determined shape of the region adjacent to the first region, wherein the predicted shape of the first region mirrors the determined shape of the region adjacent to the first region with respect to an arbitrary axis.

The processor determines a shape of the region adjacent to the first region, based on the image information of the region adjacent to the first region, and predicts a shape of the first region according to a predetermined pattern, when the determined shape of the region adjacent to the first region corresponds to the predetermined pattern.

The display displays the reconstructed region in the first reference image such that the reconstructed region is distinguished from an original region in the first reference image.

The processor reconstructs the first region within the medical image, and the display displays the reconstructed first region in the medical image such that the reconstructed first region overlaps the medical image.

When a type of the first reference image is different from a type of the medical image, the processor reconstructs the first region within the medical image and converts coordinate information of the reconstructed first region from a coordinate system of the medical image to a coordinate system of the first reference image, and the display displays the region corresponding to the object within the first reference image, based on the converted coordinate information of the reconstructed first region.

The medical image displaying apparatus further includes a user interface, wherein the display displays the obtained image information of the region adjacent to the first region and the predicted image information of the first region, the user interface receives a user input for correcting the predicted image information of the first region, and the processor corrects the predicted image information of the first region, based on the user input, reconstructs the region corresponding to the object within the first reference image, based on the corrected image information of the first region, and controls the display to display the reconstructed region.

According to an aspect of another embodiment, a non-transitory computer-readable recording medium having recorded thereon a program, which when executed by a computer, performs steps of: displaying a medical image of an object captured in real time, and a first reference image of the object registered with the medical image; obtaining image information of a region adjacent to a first region within the medical image, wherein the first region is a distorted region of the medical image with respect to a shape of the object; predicting image information of the first region, based on the image information of the region adjacent to the first region; and reconstructing a region corresponding to the object within the first reference image, based on the predicted image information of the first region, and displaying the reconstructed region.

### BRIEF DESCRIPTION OF THE DRAWINGS

These and/or other aspects will become apparent and more readily appreciated from the following description of the embodiments, taken in conjunction with the accompanying drawings in which:
FIG. 1 explains a method of displaying a medical image on a medical image displaying apparatus, according to an embodiment of the present disclosure;
FIG. 2 is a block diagram of a medical image displaying apparatus according to an embodiment;
FIG. 3 is a block diagram of a medical image displaying apparatus according to an embodiment;
FIG. 4 is a block diagram of a medical image displaying apparatus according to another embodiment;
FIG. 5 is a flowchart of a method in which a medical image displaying apparatus displays a medical image, according to an embodiment;
FIG. 6 explains a medical image displayed on a medical image displaying apparatus, according to an embodiment;
FIG. 7 explains a method of predicting a region of which an object has been shape-distorted within a medical image, according to an embodiment;
FIG. 8 is a flowchart of a method of predicting a region of which an object has been shape-distorted within a medical image, according to an embodiment;
FIGS. 9A and 9B explain a method of predicting a region of which an object has been shape-distorted within a medical image, according to an embodiment;
FIGS. 10A-10C explain a medical image displayed on a screen of a display of a medical image displaying apparatus, according to an embodiment;
FIG. 11 explains a process of correcting a predicted region according to a user input, according to an embodiment;
FIG. 12 is a block diagram of a structure of an ultrasound diagnosis apparatus according to an embodiment; and
FIG. 13 is a block diagram of a structure of a wireless probe according to an embodiment.

### DETAILED DESCRIPTION

The terms used in this specification are those general terms currently widely used in the art in consideration of functions regarding the inventive concept, but the terms may vary according to the intention of those of ordinary skill in the art, precedents, or new technology in the art. Also, some terms may be arbitrarily selected by the applicant, and in this case, the meaning of the selected terms will be described in detail in the detailed description of the present specification. Thus, the terms used in the specification should be understood not as simple names but based on the meaning of the terms and the overall description of the invention.

When a part "includes" or "comprises" an element, unless there is a particular description contrary thereto, the part can further include other elements, not excluding the other elements. Also, the term "unit" in the specification means a software component or hardware component such as a field-programmable gate array (FPGA) or an application-specific integrated circuit (ASIC), and performs a specific function. However, the term "unit" is not limited to software or hardware. The "unit" may be formed so as to be in an addressable storage medium, or may be formed so as to operate one or more processors. Thus, for example, the term "unit" may refer to components such as software components, object-oriented software components, class components, and task components, and may include processes, functions, attributes, procedures, subroutines, segments of program code, drivers, firmware, micro codes, circuits, data, a database, data structures, tables, arrays, or variables. A function provided by the components and "units" may be associated with a smaller number of components and "units", or may be divided into additional components and "units".

While such terms as "first," "second," etc., may be used to describe various components, such components are not limited thereto. These terms are used only to distinguish one component from another. For example, a first component may be referred to as a second component without departing from the scope of the inventive concept, and similarly, a second component may be referred to as a first component. As used herein, the term "and/or" includes any and all combinations of one or more of the associated listed items. Expressions such as "at least one of," when preceding a list of elements, modify the entire list of elements and do not modify the individual elements of the list.

In the present specification, an "image" may refer to multi-dimensional data composed of discrete image elements (e.g., pixels in a two-dimensional (2D) image and voxels in a three-dimensional (3D) image).

Throughout the specification, an "ultrasound image" refers to an image of an object, which is obtained using ultrasound waves. An ultrasound image may denote an image obtained by irradiating an ultrasound signal generated from a transducer of a probe to an object, and receiving information of an echo signal reflected by the object. Also, the ultrasound image may be variously implemented, and for example, the ultrasound image may be at least one of an amplitude (A) mode image, a brightness (B) mode image, a color (C) mode image, and a Doppler (D) mode image. Also, the ultrasound image may be a two-dimensional (2D) image or a three-dimensional (3D) image.

Furthermore, an "object" may be a human, an animal, or a part of a human or animal. For example, the object may be an organ (e.g., the liver, the heart, the womb, the brain, a breast, or the abdomen), a blood vessel, or a combination thereof. Also, the object may be a phantom. The phantom means a material having a density, an effective atomic number, and a volume that are approximately the same as those of an organism.

Throughout the specification, a "user" may be, but is not limited to, a medical expert, for example, a medical doctor, a nurse, a medical laboratory technologist, or a medical imaging expert, or a technician who repairs medical apparatuses.

Reference will now be made in detail to embodiments, examples of which are illustrated in the accompanying drawings. In this regard, the present embodiments may have different forms and should not be construed as being limited to the descriptions set forth herein.

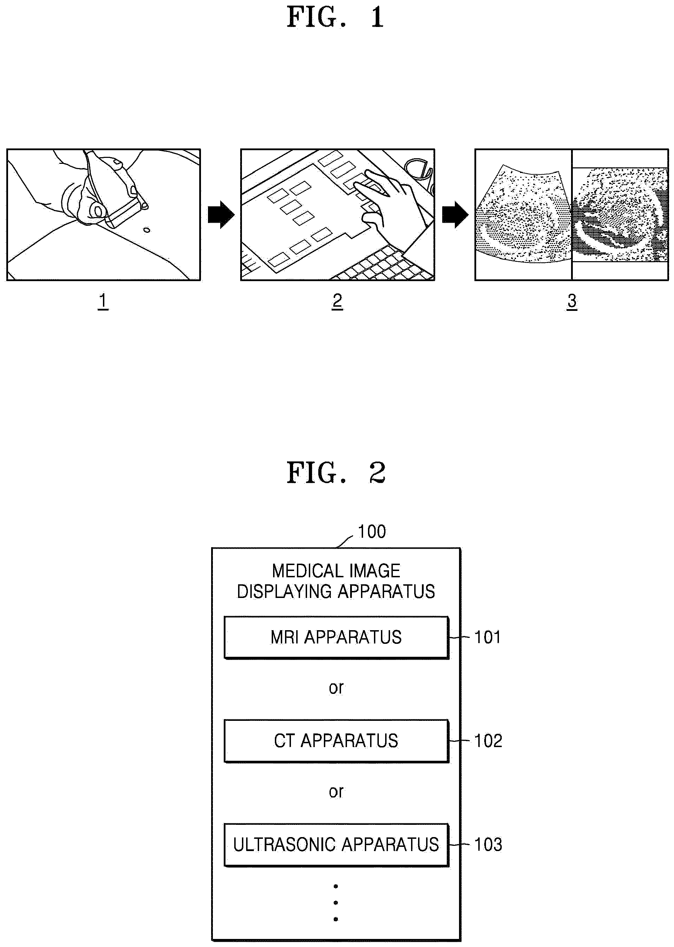

FIG. 1 explains a method of displaying a medical image on a medical image displaying apparatus, according to an embodiment of the present disclosure.

Referring to 1 of FIG. 1, the medical image displaying apparatus may obtain a medical image of an object that is a portion of the body of a patient. For example, when the medical image displaying apparatus is an ultrasound diagnosis apparatus, a user may obtain a medical image of an object by scanning the object by using the ultrasound diagnosis apparatus. The medical image displaying apparatus may obtain not only an ultrasound image but also images of various modalities. The various modality images may include an optical coherence tomography (OCT) image, a computed tomography (CT) image, a magnetic resonance (MR) image, an X-ray image, a single-photon emission computed tomography (SPECT) image, a positron emission tomography (PET) image, a C-arm image, a PET-CT image, and a PET-MR image. It will be understood by one of ordinary skill in the art that other types of images may be included.

Referring to 2 of FIG. 2, the medical image displaying apparatus may receive a user input for controlling the medical image displaying apparatus. For example, the medical image displaying apparatus may receive a user input for controlling images having different modalities to be displayed on the screen of a display.

Referring to 3 of FIG. 1, the medical image displaying apparatus may display the medical image of the object on the screen of the display. For example, the medical image displaying apparatus may display an ultrasound image of the object captured in real time, and a CT image registered with the ultrasound image. When the location, shape, and the like of the object have been changed in real time, the medical image displaying apparatus may obtain image information of a region corresponding to the object from the ultrasound image, and may reconstruct a region corresponding to the object within the CT image, based on the obtained image information. The medical image displaying apparatus may display a reconstructed CT image.

FIG. 2 is a block diagram of a medical image displaying apparatus according to an embodiment.

According to an embodiment, a medical image displaying apparatus 100 may obtain a medical image and display the medical image on a screen. For example, the medical image displaying apparatus 100 may be a magnetic resonance imaging (MRI) apparatus 101, a CT apparatus 102, an X-ray imaging apparatus (not shown), an angiography apparatus (not shown), an ultrasonic apparatus 103, or the like, but embodiments are not limited thereto.

The MRI apparatus 101 is an apparatus for obtaining a sectional image of a part of an object by expressing, in a contrast comparison, a strength of a MR signal with respect to a radio frequency (RF) signal generated in a magnetic field having a specific strength.

Since the CT apparatus 102 is capable of providing a cross-sectional image of the object, the CT apparatus 102 may express an inner structure (e.g., an organ such as a kidney, a lung, etc.) of the object without an overlap therebetween, compared to a general X-ray imaging apparatus. The CT apparatus 102 may obtain a plurality of images with a thickness not more than 2 mm for several tens to several hundreds of times per second and then may process the plurality of images, thereby providing a relatively accurate cross-sectional image of the object.

The X-ray imaging apparatus is an apparatus for imaging internal structures of a human body by transmitting an X-ray through the human body. The angiography apparatus is an apparatus that visualize the inside of blood vessels (arteries or veins) of a body by injecting a contrast agent into the blood vessels via an about 2mm-long tube, called a catheter, and imaging the blood vessels by using X-rays.

The ultrasonic apparatus 103 is an apparatus that transmits an ultrasonic signal from the surface of the body of an object toward a certain inner part of the body by using a probe and obtain an image of a cross-section of soft tissue or a blood flow image by using information about an ultrasonic signal reflected by the inner part of the body.

According to an embodiment, the medical image displaying apparatus 100 may be realized in various types. For example, the medical image displaying apparatus 100 may be implemented by using a fixed terminal or a movable terminal. Examples of the movable terminal may include a smartphone, a laptop computer, a personal digital assistant (PDA), and a tablet PC.

According to an embodiment, the medical image displaying apparatus 100 may exchange medical image data with a hospital server or another medical apparatus in a hospital, which is connected thereto via a Picture Archiving and Communication System (PACS). Also, the medical image displaying apparatus 100 may perform data communication with a server or the like, according to a Digital Imaging and Communications in Medicine (DICOM) standard.

According to an embodiment, the medical image displaying apparatus 100 may include a touch screen. The touch screen may be configured to detect not only a touch input position and a touched area but also a touch input pressure. The touch screen may be configured to detect not only a real touch but also a proximity touch.

Throughout the specification, the term "real touch" denotes a case in which a touch tool (e.g., a finger or an electronic pen) really touches a screen, and the term "proximity touch" denotes a case in which the touch tool does not actually touch the screen but approaches a position which is separated from the screen by a certain distance.

According to an embodiment, the medical image displaying apparatus 100 may sense a user's touch gesture for a medical image through the touch screen. Examples of the user's touch gesture (touch input) may include tap, touch and hold, double tap, drag, panning, flick, drag and drop, swipe, and pinch.

According to an embodiment, the medical image displaying apparatus 100 may provide a graphical user interface (GUI). The graphical user interface (GUI) may receive a portion or the entirety of a user input for selecting an object (for example, an object of interest, a region of interest, or a point of interest) from the medical image or a user input for controlling display of the medical image.

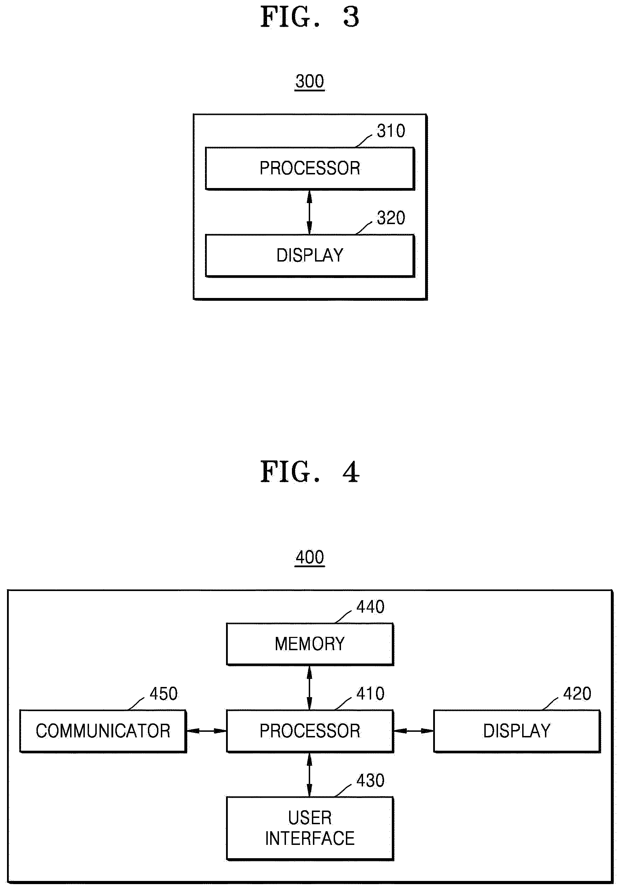

FIG. 3 is a block diagram of a medical image displaying apparatus 300 according to an embodiment.

Referring to FIG. 3, the medical image displaying apparatus 300 may include a processor 310 and a display 320. However, all of the illustrated components are not essential. The medical image displaying apparatus 300 may be implemented by more or less components than those illustrated in FIG. 3. The aforementioned components will now be described in detail.

The processor 310 may obtain a medical image of an object captured in real time (hereinafter, referred to as a medical image). The medical image of the object captured in real time may be directly obtained by the medical image displaying apparatus 300 or may be received from an external apparatus.

The processor 310 may extract image information about a feature point of the object from the medical image captured in real time. The processor 310 may display update information about the object on a reference image, based on the extracted image information about the feature point. In other words, the processor 310 may reconstruct and display a region corresponding to the object within the reference image, based on the extracted image information about the feature point. When the feature point of the object is extracted from the medical image captured in real time, a shadow and/or an artifact may exist within the region corresponding to the object, and thus the region corresponding to the object may be distorted and displayed.

The processor 310 may analyze the medical image and identify a first region in which the shape of the object is distorted. For example, the processor 310 may determine, as the first region, a region having a low image quality within the region corresponding to the object in the medical image. When the image quality of a certain region within the medical image is lower than a preset standard, the processor 310 may determine the certain region as the region having a low image quality.

The processor 310 may obtain image information of a region adjacent to the first region. For example, the image information may include, but is not limited to, contour information of an object within an image, gradient information of the object within the image, intensity information about a pixel within the image, and histogram information indicating a pixel distribution from a dark region to a bright region within the image. The gradient information may be information about an intensity of a pixel value within the image or information about a change in the pixel value. The contour information may be obtained based on an image processing filter. The image processing filter may correspond to at least one of a gabor filter, a sobel filter, and a Roberts filter, and embodiments are not limited thereto.

For example, the processor 310 may obtain the image information of the region adjacent to the first region by performing at least one of an operation of extracting a feature point of the object from the region adjacent to the first region, an operation of extracting a contour of the region adjacent to the first region, an operation of extracting variations in pixel values of the region adjacent to the first region, based on an image processing filter, an operation of extracting histogram information of the region adjacent to the first region, an operation of extracting a difference between the pixel values of the region adjacent to the first region, and an operation of extracting shape information of the region adjacent to the first region. It will be understood by one of ordinary skill in the art that the processor 310 may perform an operation based on any of the other methods to obtain the image information of the region adjacent to the first region.

When the processor 310 has difficulty in extracting the feature point of the object from the medical image, the processor 310 may extract a feature point from a portion of the region corresponding to the object, and may reconstruct a region from which no feature points are extracted, by using an image processing technique using contour information, gradient information, and the like, a modeling technique using a preset model value, a fitting technique, a machine running technique, an artificial intelligence (AI) technique, or the like.

For example, the processor 310 may predict image information of the first region, based on the image information of the region adjacent to the first region. In detail, the processor 310 may determine the shape of the region adjacent to the first region, based on the image information of the region adjacent to the first region. The processor 310 may predict the shape of the first region by mirroring the determined shape of the region adjacent to the first region with respect to an arbitrary axis. For example, the artificial axis may be an axis that passes through a reference point associated with the region adjacent to the first region. The reference point may be a center point between the first region and the region adjacent to the first region. When the determined shape of the region adjacent to the first region is formed according to a predetermined pattern, the processor 310 may predict the shape of the first region according to the pattern.

The processor 310 may reconstruct a region corresponding to the object in a first reference image, based on the predicted image information of the first region. The processor 310 may control the display 320 to display a first reference image in which the region corresponding to the object has been reconstructed.

The display 320 displays a predetermined screen image. In detail, the display 320 displays a predetermined screen image under the control of the processor 310. The display 320 includes a display panel (not shown), and may display a medical image and the like on the display panel.

The display 320 may display the medical image of the object captured in real time, and the first reference image of the object registered with the medical image. For example, the display 320 may display an ultrasound image of a liver that is captured in real time. The display 320 may display a CT image of the same liver as the liver displayed on the ultrasound image. The first reference image is an image obtained from the medical image displaying apparatus 300 or the external apparatus and stored.

The display 320 may display the first reference image in which the region corresponding to the object has been reconstructed. The display 320 may distinguish the reconstructed region in the first reference image from an original region and display them.

The processor 310 may reconstruct the first region of the medical image captured in real time. The display 320 may display the reconstructed first region of the medical image such that the reconstructed first region overlaps the medical image.

The processor 310 may reconstruct the first region of the medical image. In this case, when the type of the first reference image is different from the type of the medical image, the processor 310 may convert coordinate information of the first region from a coordinate system of the medical image to a coordinate system of the first reference image. The processor 310 may reconstruct the region corresponding to the first region within the first reference image, based on the coordinate information of the first region converted into the coordinate system of the first reference image. The display 320 may display the reconstructed first reference image.

The display 320 may magnify and display the region corresponding to the object within the medical image. The display 320 may magnify and display the reconstructed region within the first reference image.

The medical image displaying apparatus 300 may predict a region of the object distorted or lost by a shadow or an artifact in the real-time medical image, by using the feature point of the object.

The medical image displaying apparatus 300 may predict the region of the object distorted or lost in the real-time medical image, and may reconstruct the region of the object, based on a result of the prediction.

The medical image displaying apparatus 300 may reconstruct and display a reference image of the object, based on image information of the reconstructed region of the object in the real-time medical image.

The medical image displaying apparatus 300 may further include a central processor to control overall operations of the processor 310 and the display 320. The central processor may be implemented by an array of a plurality of logic gates, or by a combination of a general-use microprocessor and a memory in which a program executable by the general-use microprocessor is stored. It will also be understood by one of ordinary skill in the art to which this example pertains that the central processor may be implemented by other types of hardware.

FIG. 4 is a block diagram of a medical image displaying apparatus 400 according to another embodiment.

The medical image displaying apparatus 400 may include a processor 410, a display 420, a user interface 430, a memory 440, and a communicator 450.

The processor 410 and the display 420 of the medical image displaying apparatus 400 of FIG. 4 are respectively the same as the processor 310 and the display 320 of the medical image displaying apparatus 300 of FIG. 3, and thus repeated descriptions thereof will be omitted. The medical image displaying apparatus 400 may be implemented by more or less components than those illustrated in FIG. 4. The aforementioned components will now be described in detail.

The user interface 430 refers to a device via which a user inputs data for controlling the medical image displaying apparatus 400. The processor 410 may control the display 430 to generate and display a screen image of the user interface 430 for receiving a command or data from the user. The display 420 may display image information of a region adjacent to a first region of which object shape has been distorted, and predicted image information of the first region. The display 420 may display, on a panel, a screen image that receives a user input for correcting the predicted image information of the first region.

The processor 410 may correct the predicted image information of the first region, based on the user input, and reconstruct a region corresponding to the object within a first reference image, based on the corrected image information of the first region. The display 420 may display a reconstructed first reference image.

The memory 440 may store data related with the object (for example, a medical image of the object, medical image data, scan-related data, and diagnosis data of a patient regarding the object) and data transmitted from an external apparatus to the medical image displaying apparatus 400. The data transmitted by the external apparatus to the medical image displaying apparatus 400 may include patient-related information, data necessary for diagnosing and treating patients, histories of previous treatments of patients, and a medical worklist (MWL) corresponding to diagnosis instructions for patients, and the like.

The communicator 450 may receive data from the external apparatus and/or transmit data to the external apparatus. For example, the communicator 450 may provide a reconstructed image of the object to an external terminal. The external terminal may be a terminal of a patient or a terminal of a doctor. The external apparatus may be a server that manages medical records of patients or an intermediate server of an application that provides information of patients. The communicator 450 may be connected to a wireless probe or the external apparatus via a Wi-Fi or Wi-Fi direct (WFD) communication network. In detail, examples of a wireless communication network via which the communicator 450 can be connected to the wireless probe or the external apparatus may include, but are not limited to, wireless LAN, Wi-Fi, Bluetooth, ZigBee, WFD, ultra wideband (UWB), infrared Data Association (IrDA), Bluetooth low energy (BLE), and near field communication (NFC).

The medical image displaying apparatus 400 may further include a central process to control overall operations of the processor 410, the display 420, the user interface 430, the memory 440, and the communicator 450. The central processor may be implemented by an array of a plurality of logic gates, or by a combination of a general-use microprocessor and a memory in which a program executable by the general-use microprocessor is stored. It will also be understood by one of ordinary skill in the art to which this example pertains that the central processor may be implemented by other types of hardware.

Various operations or applications that the medical image displaying apparatuses 300 and 400 execute will now be described. However, matters to be clearly understood and expected by one of ordinary skill in the art to which the present invention pertains may be understood by typical implementations even when none of the processors 310 and 410, the displays 320 and 420, the user interface 430, the memory 440, and the communicator 450 is specified, and the scope of the present invention is not limited by the titles or physical/logical structures of specified components.

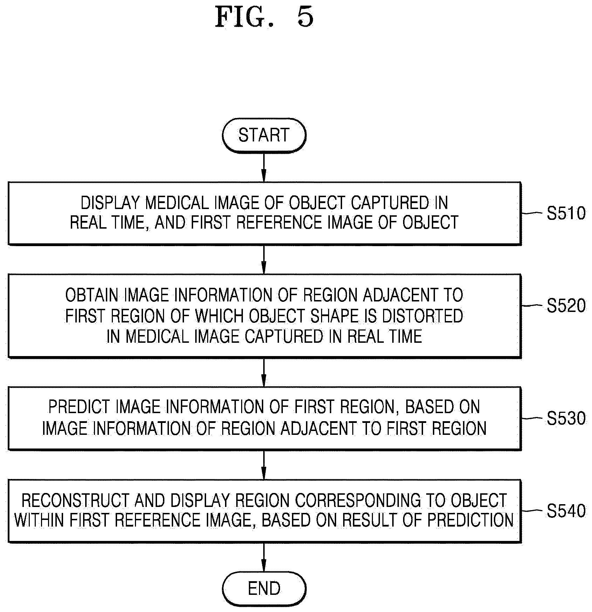

FIG. 5 is a flowchart of a method in which a medical image displaying apparatus displays a medical image, according to an embodiment.

In operation S510 of FIG. 5, the medical image displaying apparatus may display a medical image of an object captured in real time, and a first reference image of the object registered with the medical image.

In operation S520, the medical image displaying apparatus may obtain image information of a region adjacent to a first region of which object shape is distorted in the medical image captured in real time. In detail, the medical image displaying apparatus may determine, as the first region, a region having a lower image quality than a preset standard within a region corresponding to the object in the medical image captured in real time. The medical image displaying apparatus may obtain image information of the region adjacent to the first region.

The medical image displaying apparatus may obtain the image information of the region adjacent to the first region by performing at least one of an operation of extracting a feature point of the object from the region adjacent to the first region, an operation of extracting a contour of the region adjacent to the first region, an operation of extracting variations in pixel values of the region adjacent to the first region, based on an image processing filter, an operation of extracting histogram information of the region adjacent to the first region, an operation of extracting a difference between the pixel values of the region adjacent to the first region, and an operation of extracting shape information of the region adjacent to the first region.

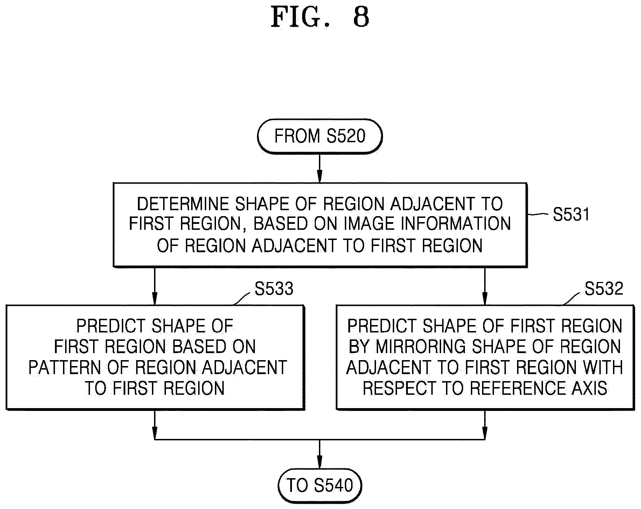

In operation S530, the medical image displaying apparatus may predict image information of the first region, based on the image information of the region adjacent to the first region. The image information may include, but is not limited to, contour information of the first region, gradient information of the first region, intensity information about a pixel within the first region, and histogram information indicating a pixel distribution from a dark region to a bright region within the first region. A method of predicting the image information of the first region will be described later with reference to FIG. 8.

In operation S540, the medical image displaying apparatus may reconstruct and display a region corresponding to the object within the first reference image, based on a result of the prediction.

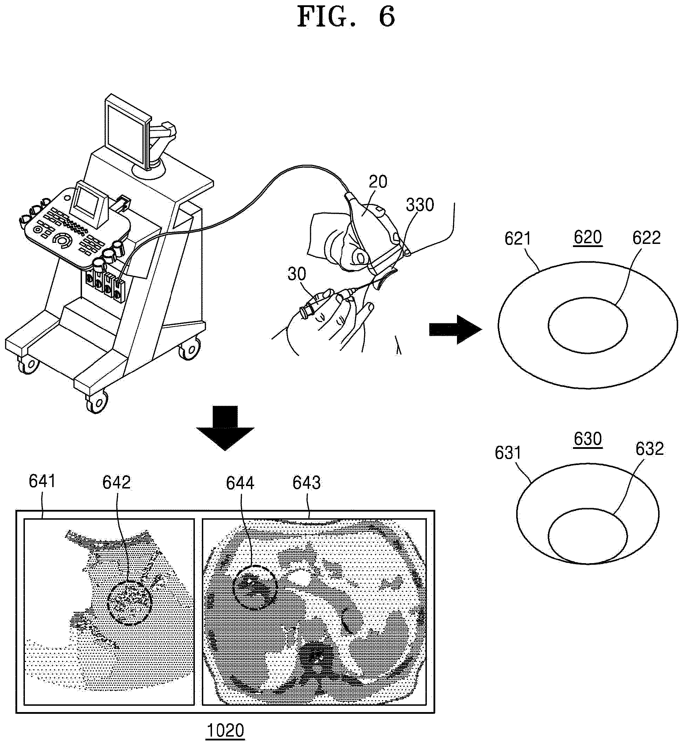

FIG. 6 explains a medical image displayed on a medical image displaying apparatus, according to an embodiment.

As shown in FIG. 6, a user may treat an object which is a portion of the body of a patient, by using an ultrasound diagnosis apparatus.

For example, to remove a tumor from the object, the user may excise a region including the tumor and a region adjacent to the region including the tumor. As shown in reference numeral 620 of FIG. 6, the user may excise a region 621 having the tumor and a region 622 adjacent to the region 621 having the tumor, by using the ultrasound diagnosis apparatus. However, when a region 631 having a tumor and a region 632 adjacent to the region 631 having the tumor are excised as shown in reference numeral 630 of FIG. 6, tumor excision needs to be performed again.

The user may treat the object by inserting a needle to the object. For example, the user may perform thermotherapy to apply heat to the object or cooling treatment to cool the object. It will be understood by one of ordinary skill in the art that the user may treat the object according to any of the other treating methods.

Referring to reference numeral 640 of FIG. 6, the ultrasound diagnosis apparatus may display a treatment process of the object on an ultrasound image 641 by photographing the object in real time. The ultrasound image 641, which is captured in real time, may magnify and display a region of the object into which a needle has been inserted. The ultrasound diagnosis apparatus may display a first reference image of the object that is registered with the ultrasound image 641. As shown in reference numeral 640 of FIG. 6, the first reference image may be a CT image 643. The CT image 643 may include a region 644 representing the object.

A region 642 representing the object within the ultrasound image 641 may be displayed inaccurately (e.g., distortedly displayed) due to a shadow region of the needle inserted into the object. For example, when the ultrasound diagnosis apparatus displays the region including the tumor, the tumor may be hidden by the shadow region of the needle. Moreover, the ultrasound diagnosis apparatus may not display accurately the region 642 of the object due to a defect of the ultrasound image 641. Accordingly, when the ultrasound diagnosis apparatus displays the ultrasound image 641 captured in real time, the ultrasound diagnosis apparatus may provide an indefective ultrasound image so that the user has no difficulty in treating the object.

The ultrasound diagnosis apparatus may obtain image information of a region adjacent to a region in which the shape of the object has been distorted. The ultrasound diagnosis apparatus may predict image information of the distorted region, based on the image information of the region adjacent to the distorted region. The ultrasound diagnosis apparatus may reconstruct and display the distorted region. For example, the ultrasound diagnosis apparatus may predict image information of a distorted region in the ultrasound image 641, and reconstruct and display the region 644 representing the object within the CT image 643, based on a result of the prediction.

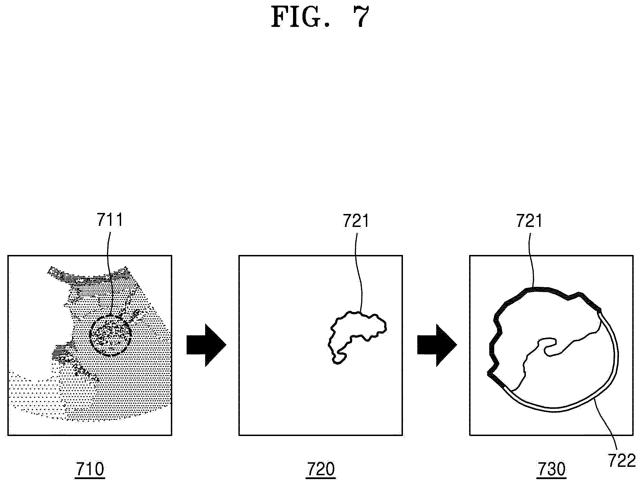

FIG. 7 explains a method of predicting a region of which an object has been shape-distorted within a medical image, according to an embodiment.

As shown in reference number 710 of FIG. 7, a medical image displaying apparatus may photograph the object in real time and display a medical image of the object on the screen of a display. While the object is being treated by external equipment such as a medical instrument, a region 711 representing the object may be distorted and displayed within the medical image, due to a shadow of the external equipment or a defect of the medical image.

For example, when radio frequency ablation (RFA) in which external equipment is inserted into a malignant tumor of a liver and excises the malignant tumor by using the heat generated by radio frequency is performed, the medical image displaying apparatus may obtain an ultrasound image of the malignant tumor captured in real time. Because of the external equipment, a shadow and/or an artifact may exist in a region corresponding to the malignant tumor and a region adjacent to the region of the malignant tumor in the ultrasound image captured in real time. Thus, the region of the malignant tumor and the region adjacent to the region of the malignant tumor may be distorted and displayed.

As shown in reference numeral 720 of FIG. 7, the medical image displaying apparatus may determine, as a first region, a region having a low image quality in a region 711 corresponding to the object within the medical image, and may obtain image information of a region 721 adjacent to the first region. The medical image displaying apparatus may obtain the image information of the region 721 by extracting a feature point of the object from the region 721 adjacent to the first region, extracting a brightness value of the region 721 adjacent to the first region, or extracting shape information of the region 721 adjacent to the first region.

As shown in reference numeral 710 of FIG. 7, during RFA with respect to the malignant tumor of the liver, a lower end portion of the region corresponding to the malignant tumor within the ultrasound image may be lost due to a shadow. In this case, the medical image displaying apparatus may extract a bright region compared with the other regions from the ultrasound image. The bright region may be a region from which the malignant tumor is excised, and may be used as an index that indicates the degree of a progression of the RFA.

As shown in reference numeral 730 of FIG. 7, the medical image displaying apparatus may determine the shape of the region 721 adjacent to the first region, based on the image information of the region 721 adjacent to the first region. The medical image displaying apparatus may predict a shape 722 of the first region, based on the determined shape of the region 721 adjacent to the first region.

For example, the medical image displaying apparatus may determine a shape of an upper end portion of the region corresponding to the malignant tumor within the ultrasound image, based on image information of the upper end portion. The image information of the upper end portion may include at least one selected from contour information, gradient information, intensity information, and histogram information. The medical image displaying apparatus may predict a shape of the lower end portion corresponding to the malignant tumor, which has been distorted or lost by a shadow or an artifact, based on the shape of the upper end portion.

FIG. 8 is a flowchart of a method of predicting a region of which an object has been shape-distorted within a medical image, according to an embodiment.

Referring to FIG. 8, in operation S531, the medical image displaying apparatus may determine a shape of the region adjacent to the first region, based on the image information of the region adjacent to the first region. The medical image displaying apparatus may perform operation S531 and may perform operation S532 or S533. The method of predicting the shape of the first region disclosed in operation S532 or S533 is merely an embodiment, and the shape of the first region may be predicted according to any of the other methods.

In operation S532, the medical image displaying apparatus may predict the shape of the first region by mirroring the shape of the region adjacent to the first region with respect to the reference axis.

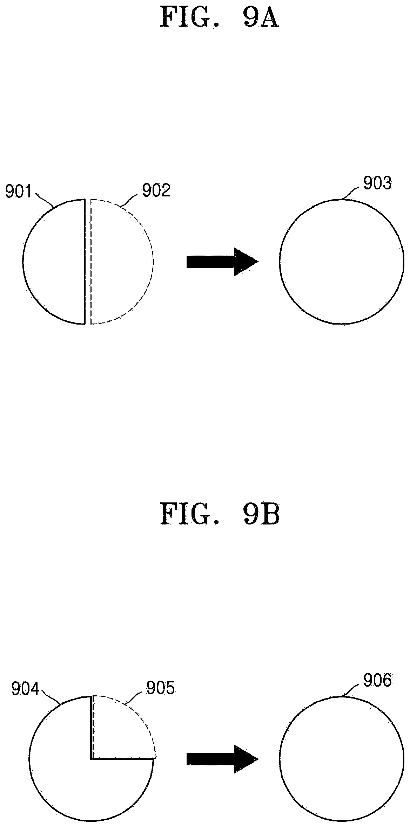

For example, FIG. 9A explains a method of predicting a region of which an object has been shape-distorted within a medical image. Referring to FIG. 9A, the medical image displaying apparatus may detect a first region 902 having a low image quality from a region (901 and 902) corresponding to an object. The medical image displaying apparatus may determine a region 901 adjacent to the first region 902 and obtain image information of the region 901 adjacent to the first region 902. The medical image displaying apparatus may predict a shape of the first region 902 by mirroring a shape of the region 901 adjacent to the first region 902 with respect to a vertical axis.

Referring back to FIG. 8, in operation S533, the medical image displaying apparatus may predict a shape of the region adjacent to the first region, according to a pattern of the region adjacent to the first region.

When the shape of the region adjacent to the first region is formed according to a predetermined pattern, the medical image displaying apparatus may predict the shape of the first region according to the pattern. The predetermined pattern may mean a certain shape, a certain model, or a certain form. Although a predetermined shape, model, or form is not a certain shape, model, or form, if a certain shape, model, or form is predicted, the certain shape, model, or form may be considered a predetermined pattern.

For example, FIG. 9B explains a method of predicting a region of which an object has been shape-distorted within a medical image. Referring to FIG. 9B, the medical image displaying apparatus may detect a first region 905 having a low image quality from a region (904 and 905) corresponding to an object. The medical image displaying apparatus may determine a region 904 adjacent to the first region 905 and obtain image information of the region 904 adjacent to the first region 905. In this case, as shown in FIG. 9B, the region 904 adjacent to the first region 905 may be obtained by combining three circular sectors each having a central angle of 90 degrees. The medical image displaying apparatus may predict the shape of the first region 905 as being circular and having a central angle of 90 degrees.

The medical image displaying apparatus may predict the image information of the first region by performing operation S532 or S533. The medical image displaying apparatus may reconstruct and display the region corresponding to the object within the first reference image, based on a result of the prediction.

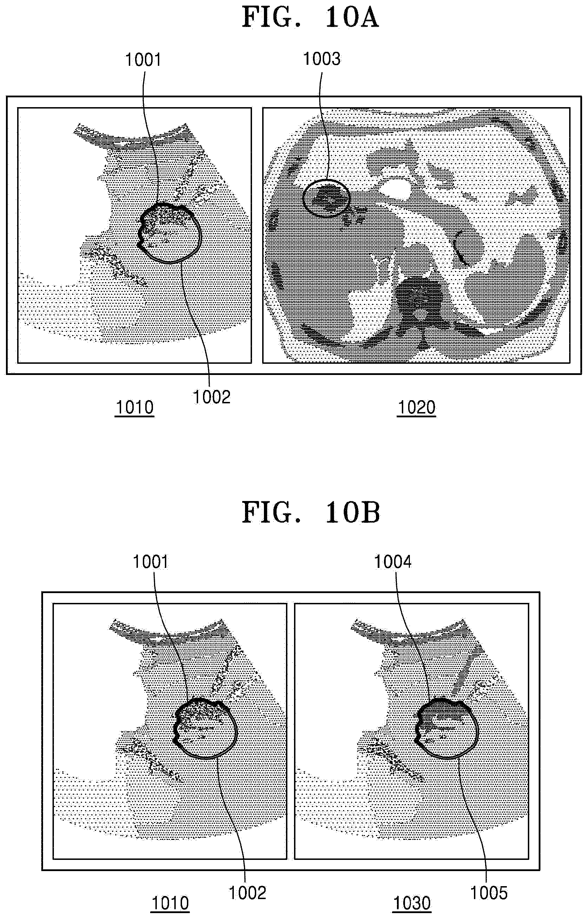

FIG. 10A explains a medical image displayed on a screen of a display of a medical image displaying apparatus, according to an embodiment.

The medical image displaying apparatus may display an ultrasound image 1010 of an object captured in real time, on the screen of the display. The medical image displaying apparatus may also display, on the screen of the display, a CT image 1020 being a first reference image of the object registered with the ultrasound image 1010, together with the ultrasound image 1010. The ultrasound image 1010 may include regions 1001 and 1002 representing the object.

A user may perform thermotherapy or cooling treatment by using an external apparatus in order to treat an object, which is a portion of the body of a patient. In this case, the regions 1001 and 1002 representing the object may be distorted by the external apparatus and displayed on the ultrasound image 1010 captured in real time. Accordingly, the medical image displaying apparatus may reconstruct and display a distorted region.

As shown in the ultrasound image 1010 of FIG. 10A, the medical image displaying apparatus may display a distorted region 1002 and a non-distorted region 1001 in the real-time ultrasound image 1010 of the object under RFA such that the distorted region 1002 is distinguished from the non-distorted region 1001 . The medical image displaying apparatus may predict image information of the distorted region 1002, based on image information of the non-distorted region 1001. The medical image displaying apparatus may reconstruct and display a region 1003 corresponding to the object within the CT image registered with the ultrasound image 1010. In other words, the medical image displaying apparatus may reconstruct and display the region 1003 corresponding to the object within the CT image 1020, which is a reference image, and may obtain a result of RTA with respect to the object based on the reconstructed region 1003, and determine whether to perform additional RFA, based on the reconstructed region 1003.

FIG. 10B explains a medical image displayed on a screen of a display of a medical image displaying apparatus, according to another embodiment.

The medical image displaying apparatus may display an ultrasound image 1010 of an object captured in real time, on the screen of the display. The medical image displaying apparatus may also display, on the screen of the display, a first ultrasound image 1030 being a first reference image of the object registered with the ultrasound image 1010, together with the ultrasound image 1010. The ultrasound image 1010 may include regions 1001 and 1002 representing the object.

The first ultrasound image 1030 being the first reference image includes a three-dimensional (3D) ultrasound image and a two-dimensional (2D) ultrasound image. A Doppler image may be at least one of a color Doppler image, a power Doppler image, a tissue Doppler image, a vector Doppler image, and a spectral Doppler image, but embodiments are not limited thereto.

As shown in FIG. 10A, the medical image displaying apparatus may predict image information of the distorted region 1002, based on image information of the non-distorted region 1001. The medical image displaying apparatus may reconstruct and display regions 1004 and 1005 corresponding to the object within the first ultrasound image 1030 registered with the ultrasound image 1010.

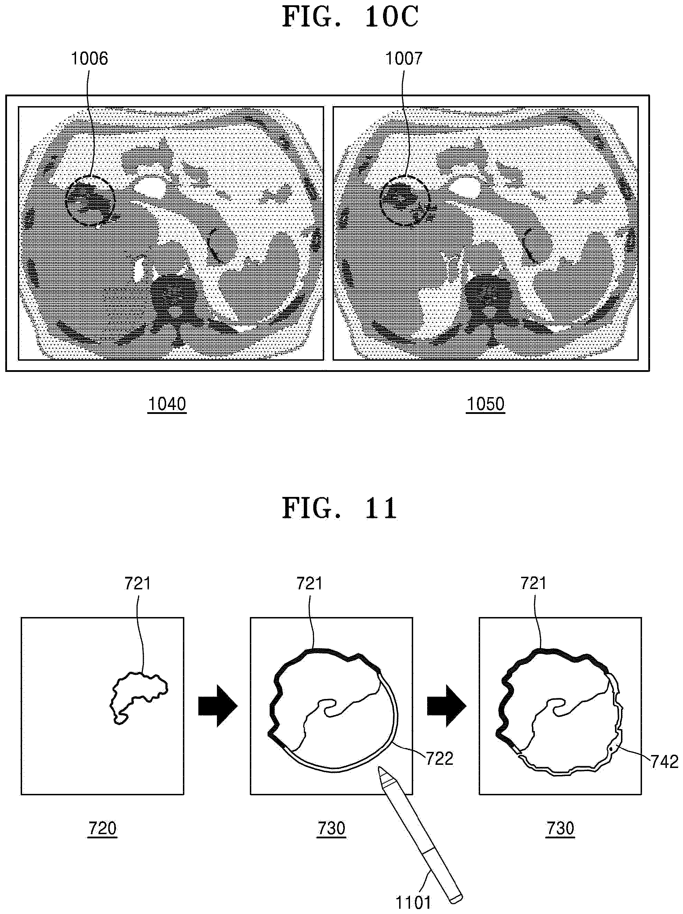

FIG. 10C explains a medical image displayed on a screen of a display of a medical image displaying apparatus, according to another embodiment.

The medical image displaying apparatus may display both a first CT image 1040 of an object 1006 including a tumor and a second CT image 1050 of an object 1007 from which tumor has been removed.

FIG. 11 explains a process of correcting a predicted region according to a user input, according to an embodiment.

As shown in reference numeral 720 of FIG. 7, a medical image displaying apparatus may determine, as the first region, the region having the low image quality in the region 711 of FIG. 7 corresponding to an object within the medical image, and may obtain the image information of the region 721 adjacent to the first region.

As shown in reference numeral 730 of FIG. 7, the medical image displaying apparatus may determine the shape of the region 721 adjacent to the first region, based on the image information of the region 721 adjacent to the first region. The medical image displaying apparatus may predict the shape 722 of the first region, based on the determined shape of the region 721 adjacent to the first region.

The medical image displaying apparatus may reconstruct the predicted shape 722 of the first region, based on the region 721 adjacent to the first region. The medical image displaying apparatus may receive a user input 1101 for correcting the predicted shape 722 of the first region, via a user interface.

The user interface refers to a device via which a user inputs data for controlling the medical image displaying apparatus. The user interface may include a display for displaying a user interface (UI) screen image for receiving a certain command or data from a user, independently from a display for displaying a medical image. The user interface may include, but is not limited to, hardware structures such as a keypad, a mouse, a touch panel, a touch screen, a track ball, and a jog switch, in addition to the display for displaying a UI screen image. The user interface may further include various input units, such as a voice recognition sensor, a gesture recognition sensor, a fingerprint recognition sensor, an iris recognition sensor, a depth sensor, and a distance sensor.

As shown in reference numeral 740 of FIG. 11, the medical image displaying apparatus may correct the shape 722 of the first region, based on the user input 1101. The medical image displaying apparatus may reconstruct the region corresponding to the object by reflecting the shape of a corrected first region in the first reference image.

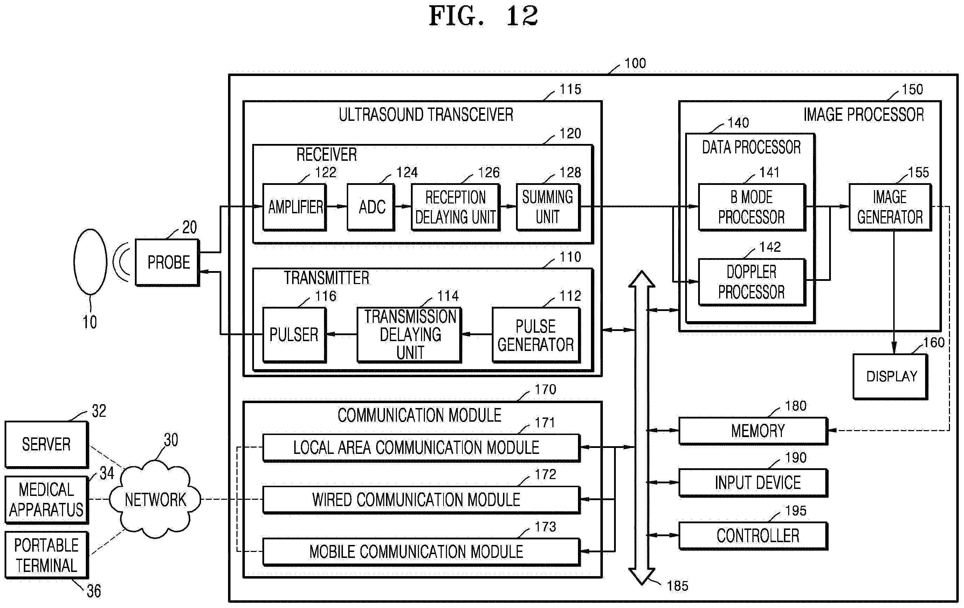

FIG. 12 is a block diagram showing a configuration of an ultrasound diagnosis apparatus 100 according to an embodiment.

Referring to FIG. 12, the ultrasound diagnosis apparatus 100 may include a probe 20, an ultrasound transceiver 115, an image processor 150, a display 160, a communication module 170, a memory 180, a user input unit 190, and a controller 195. Also, the above-mentioned components may be connected with each other via a bus 185, and the image processor 150 may include an image generator 155, a cross-section information detector 130, and a display 160.

A person of ordinary skill in the art will understand that other general purpose components besides the components illustrated in FIG. 12 may be further included.

In some embodiments, the ultrasound diagnosis apparatus 100 may be a cart type apparatus or a portable type apparatus. Examples of portable ultrasound diagnosis apparatuses may include, but are not limited to, a picture archiving and communication system (PACS) viewer, a smartphone, a laptop computer, a personal digital assistant (PDA), and a tablet PC.

The probe 20 includes a plurality of transducers, and the plurality of transducers oscillate in response to electric signals and generate acoustic energy, that is, ultrasound waves. Furthermore, the probe 20 may be connected to the main body of the ultrasound diagnosis apparatus 100 by wire or wirelessly, and according to embodiments, the ultrasound diagnosis apparatus 100 may include a plurality of probes 20.

A transmitter 110 supplies a driving signal to the probe 20. The transmitter 110 includes a pulse generator 112, a transmission delaying unit 114, and a pulser 116. The pulse generator 112 generates pulses for forming transmission ultrasound waves based on a predetermined pulse repetition frequency (PRF), and the transmission delaying unit 114 delays the pulses by delay times necessary for determining transmission directionality. The pulses which have been delayed correspond to a plurality of piezoelectric vibrators included in the probe 20, respectively. The pulser 116 applies a driving signal (or a driving pulse) to the probe 20 based on timing corresponding to each of the pulses which have been delayed.

A receiver 120 generates ultrasound data by processing echo signals received from the probe 20. The receiver 120 may include an amplifier 122, an analog-to-digital converter (ADC) 124, a reception delaying unit 126, and a summing unit 128. The amplifier 122 amplifies echo signals in each channel, and the ADC 124 performs analog-to-digital conversion with respect to the amplified echo signals. The reception delaying unit 126 delays digital echo signals output by the ADC 124 by delay times necessary for determining reception directionality, and the summing unit 128 generates ultrasound data by summing the echo signals processed by the reception delaying unit 126.

The image processor 150 generates an ultrasound image by scan-converting ultrasound data generated by the ultrasound transceiver 115.

The ultrasound image may be not only a grayscale ultrasound image obtained by scanning an object in an amplitude (A) mode, a brightness (B) mode, and a motion (M) mode, but also may represent motion of an object by using a Doppler image. The Doppler image may be a blood flow Doppler image showing flow of blood (also referred to as a color Doppler image), a tissue Doppler image showing a movement of tissue, or a spectral Doppler image showing a moving speed of an object as a waveform.

A B mode processor 141 extracts B mode components from ultrasound data and processes the B mode components. An image generator 155 may generate an ultrasound image indicating signal intensities as brightness based on the extracted B mode components 141.

Similarly, a Doppler processor 142 may extract Doppler components from ultrasound data, and the image generator 155 may generate a Doppler image indicating a movement of an object as colors or waveforms based on the extracted Doppler components.

The image generator 155 may generate a 2D ultrasound image or a 3D ultrasound image of an object, and may also generate an elastic image that shows a degree of deformation of an object 10 depending on pressure. Furthermore, the image generator 155 may display various pieces of additional information in an ultrasound image by using text and graphics. In addition, the generated ultrasound image may be stored in the memory 180.

A display 160 displays the generated ultrasound image. The display 160 may display not only an ultrasound image, but also various pieces of information processed by the ultrasound diagnosis apparatus 100 on a screen image via a graphical user interface (GUI). In addition, the ultrasound diagnosis apparatus 100 may include two or more displays 160 according to embodiments.

The display 160 may include at least one of a liquid crystal display (LCD), a thin film transistor-liquid crystal display (TFT-LCD), an organic light-emitting diode (OLED), a flexible display, a 3D display, and an electrophoretic display.

Also, in the case where the display 160 and a user input unit configure a touchscreen by forming a layered structure, the display 160 may be used as not only an output unit but also an input unit that may receive information via a user's touch.

The touchscreen may be configured to detect even a touch pressure as well as a touch location and a touched area. Also, the touchscreen may be configured to detect not only a real-touch but also a proximity touch.

In the specification, a "real-touch" denotes a case where a pointer is actually touched onto a screen, and a "proximity-touch" denotes a case where a pointer does not actually touch a screen but is held apart from a screen by a predetermined distance. In the specification, a pointer denotes a touch tool for touching or proximity-touching a specific portion of a displayed screen. For example, a pointer may be an electronic pen, a finger, etc.

Although not shown in the drawings, the ultrasound diagnosis apparatus 100 may include various sensors inside or in the vicinity of a touchscreen in order to detect a direct touch or a proximity touch with respect to the touchscreen. An example of a sensor for detecting a touch with respect to the touchscreen includes a tactile sensor.

The tactile sensor denotes a sensor for detecting a contact of a specific object to a degree felt by a person or more. The tactile sensor may detect various information such as roughness of a contact surface, hardness of a contact object, and the temperature of a contact point.

Also, an example of a sensor for detecting a touch with respect to the touchscreen includes a proximity sensor. The proximity sensor denotes a sensor for detecting an object approaching a predetermined detection surface or the existence of an object in the neighborhood by using an electromagnetic force or an infrared ray without any mechanical contact.

An example of a proximity sensor includes a transmissive photoelectric sensor, a direct reflective photoelectric sensor, a mirror reflective photoelectric sensor, a high frequency oscillation type proximity sensor, a capacitive proximity sensor, a magnetic proximity sensor, an infrared proximity sensor, etc.

The communication module 170 is connected to a network 30 by wire or wirelessly to communicate with an external device or a server. The communication module 170 may exchange data with a hospital server or another medical apparatus in a hospital, which is connected thereto via a PACS. Furthermore, the communication module 170 may perform data communication according to the digital imaging and communications in medicine (DICOM) standard.

The communication module 170 may transmit or receive data related to diagnosis of an object, e.g., an ultrasound image, ultrasound data, and Doppler data of the object, via the network 30 and may also transmit or receive medical images captured by another medical apparatus, e.g., a computed tomography (CT) apparatus, a magnetic resonance imaging (MRI) apparatus, or an X-ray apparatus. Furthermore, the communication module 170 may receive information about a diagnosis history or medical treatment schedule of a patient from a server and utilizes the received information to diagnose the patient. Furthermore, the communication module 170 may perform data communication not only with a server or a medical apparatus in a hospital, but also with a portable terminal of a medical doctor or patient.

The communication module 170 is connected to the network 30 by wire or wirelessly to exchange data with a server 32, a medical apparatus 34, or a portable terminal 36. The communication module 170 may include one or more components for communication with external devices. For example, the communication module 1300 may include a local area communication module 171, a wired communication module 172, and a mobile communication module 173.

The local area communication module 171 refers to a module for local area communication within a predetermined distance. Examples of local area communication techniques according to an embodiment may include, but are not limited to, wireless LAN, Wi-Fi, Bluetooth, ZigBee, Wi-Fi Direct (WFD), ultra wideband (UWB), infrared data association (IrDA), Bluetooth low energy (BLE), and near field communication (NFC).

The wired communication module 172 refers to a module for communication using electric signals or optical signals. Examples of wired communication techniques according to an embodiment may include communication via a twisted pair cable, a coaxial cable, an optical fiber cable, and an Ethernet cable.

The mobile communication module 173 transmits or receives wireless signals to or from at least one selected from a base station, an external terminal, and a server on a mobile communication network. The wireless signals may be voice call signals, video call signals, or various types of data for transmission and reception of text/multimedia messages.

The memory 180 stores various data processed by the ultrasound diagnosis apparatus 100. For example, the memory 180 may store medical data related to diagnosis of an object, such as ultrasound data and an ultrasound image that are input or output, and may also store algorithms or programs which are to be executed in the ultrasound diagnosis apparatus 100.

The memory 180 may be any of various storage media, e.g., a flash memory, a hard disk drive, EEPROM, etc.

Furthermore, the ultrasound diagnosis apparatus 100 may utilize web storage or a cloud server that performs the storage function of the memory 180 online.

The user input unit 190 generates input data which a user inputs in order to control an operation of the ultrasound diagnosis apparatus 100. The user input unit 190 may include a hardware configuration such as a keypad, a mouse, a touchpad, a track ball, and a jog switch, but is not limited thereto, and may further include various configurations such as an electrocardiogram measurement module, a breathing measurement module, a voice recognition sensor, a gesture recognition sensor, a fingerprint recognition sensor, an iris recognition sensor, a depth sensor, and a distance sensor.

Particularly, the user input unit 190 may also include a touchscreen in which a touchpad and the display 160 form a layered structure.

In this case, the ultrasound diagnosis apparatus 100 according to an embodiment may display an ultrasound image of a predetermined mode and a control panel for an ultrasound image on the touchscreen. Also, the ultrasound diagnosis apparatus 100 may detect a user's touch gesture for an ultrasound image via the touchscreen.

The ultrasound diagnosis apparatus 100 according to an embodiment may physically include some buttons frequently used by a user from among buttons included in a control panel of a general ultrasound apparatus, and provide the rest of the buttons in the form of a graphical user interface (GUI) via the touchscreen.

The controller 195 may control all operations of the ultrasound diagnosis apparatus 100. In other words, the controller 195 may control operations among the probe 20, the ultrasound transceiver 100, the image processor 150, the communication module 170, the memory 180, and the user input unit 190 shown in FIG. 1.

All or some of the probe 20, the ultrasound transceiver 115, the image processor 150, the communication module 170, the memory 180, the user input unit 190, and the controller 195 may be implemented as software modules. However, embodiments of the present invention are not limited thereto, and some of the components stated above may be implemented as hardware modules. Furthermore, at least one selected from the ultrasound transceiver 115, the image processor 150, and the communication module 170 may be included in the controller 195. However, embodiments of the present invention are not limited thereto.

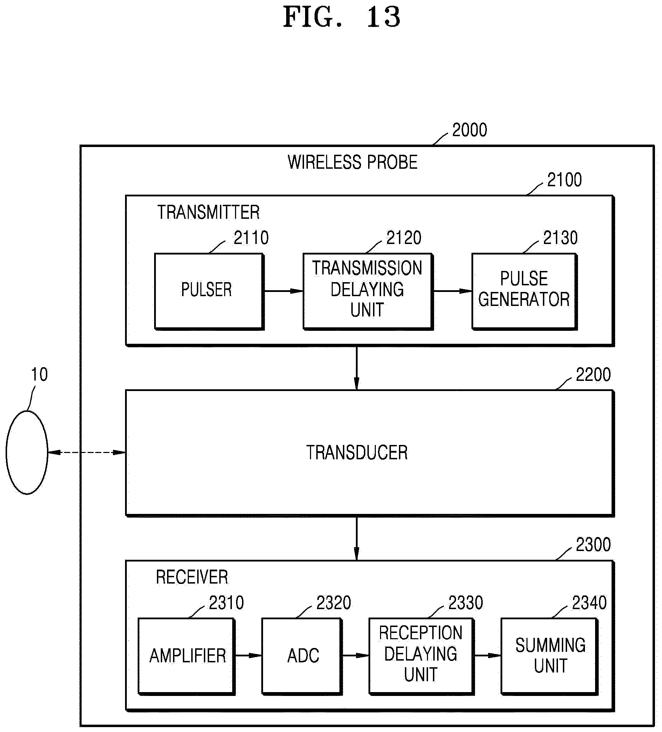

FIG. 13 is a block diagram showing a configuration of a wireless probe 2000 according to an embodiment. As described above with reference to FIG. 1, the wireless probe 2000 may include a plurality of transducers, and, according to embodiments, may include some or all of the components of the ultrasound transceiver 100 shown in FIG. 1.

The wireless probe 2000 according to the embodiment shown in FIG. 13 includes a transmitter 2100, a transducer 2200, and a receiver 2300. Since descriptions thereof are given above with reference to FIG. 1, detailed descriptions thereof will be omitted here. In addition, according to embodiments, the wireless probe 2000 may selectively include a reception delaying unit 2330 and a summing unit 2340.

The wireless probe 2000 may transmit ultrasound signals to the object 10, receive echo signals from the object 10, generate ultrasound data, and wirelessly transmit the ultrasound data to the ultrasound diagnosis apparatus 1000 shown in FIG. 1.

The wireless probe 2000 may be a smart apparatus that may perform ultrasound scanning by including a transducer array. Specifically, the wireless probe 2000 is a smart apparatus, scans an object by using a transducer array, and obtains ultrasound data. Then, the wireless probe 2000 may generate and/or display an ultrasound image by using the obtained ultrasound data. The wireless probe 2000 may include a display, and display a screen including a user interface screen for controlling at least one ultrasound image and/or a scan operation of an object via the display.

While a user scans a patient's predetermined body portion, which is an object, by using the wireless probe 2000, the wireless probe 2000 and the ultrasound diagnosis apparatus 100 may continue to transmit/receive predetermined data via a wireless network. Specifically, while a user scans a patient's predetermined body portion, which is an object, by using the wireless probe 2000, the wireless probe 2000 may transmit ultrasound data to the ultrasound diagnosis apparatus 100 in real-time via the wireless network. The ultrasound data may be updated in real-time and transmitted from the wireless probe 2000 to the ultrasound diagnosis apparatus 100 as ultrasound scanning is performed continuously.

The above-described ultrasound diagnosis apparatus may be implemented by using a hardware component, a software component, and/or a combination of a hardware component and a software component. For example, the apparatus and the component described in the exemplary embodiments may be implemented by using one or more general-purpose computers or a special-purpose computers such as, for example, a processor, a controller, an arithmetic logic unit (ALU), a digital signal processor, a microcomputer, a field programmable array (FPA), a programmable logic unit (PLU), a microprocessor, or any device that may execute an instruction and respond thereto.

A processor may execute an operating system (OS) and one or more software applications executed on the OS. Also, the processor may access, store, manipulate, process, and generate data in response to execution of software.

For convenience of understanding, though description has been made to the case where one processor is used, a person of ordinary skill in the art will understand that the processor may include a plurality of processing elements and/or processing elements having a plurality of types. For example, the processor may include a plurality of processors, or one processor and one controller. Also, the processor may include a different processing configuration such as a parallel processor.

Software may include a computer program, a code, an instruction, or a combination of one or more of these, and configure the processor to operate as desired, or instruct the processor independently or collectively.

Software and/or data may be embodied permanently or temporarily in a certain type of a machine, a component, a physical device, virtual equipment, a computer storage medium or device, or a transmitted signal wave in order to allow the processor to analyze the software and/or data, or to provide an instruction or data to the processor. Software may be distributed on a computer system connected via a network, and stored and executed in a distributed fashion. Software and data may be stored in one or more non-transitory computer-readable recording media.

The methods according to exemplary embodiments may be embodied in the form of program commands executable through various computer means, which may be recorded on a non-transitory computer-readable recording medium. The non-transitory computer-readable recording medium may include program commands, data files, and data structures either alone or in combination. The program commands recorded on the non-transitory computer-readable recording medium may be those that are especially designed and configured for the inventive concept, or may be those that are known and available to computer programmers skilled in the art.

Examples of the non-transitory computer-readable recording medium include magnetic recording media such as hard disks, floppy disks, and magnetic tapes, optical recording media such as CD-ROMs and DVDs, magneto-optical recording media such as floptical disks, and hardware devices such as ROMs, RAMs, and flash memories that are especially configured to store and execute program commands.

Examples of the program commands include machine language codes that may be generated by a compiler, and high-level language codes that may be executed by a computer by using an interpreter.

The above hardware device may be configured to operate as one or more software modules in order to perform an operation of an exemplary embodiment, and vice versa.

Though the exemplary embodiments have been described by a limited number of exemplary embodiments and drawings, a person of ordinary skill in the art will make various modifications and changes from the above exemplary embodiments. For example, even when the described technologies are performed in an order different from the described method and/or components such as the described system, structure, apparatus, and circuit are coupled or combined in a form different from the described method, or replaced by other components or equivalents thereof, a proper result may be accomplished.

Therefore, the scope of the inventive concept should not be limited and determined by the described exemplary embodiments, but should be determined by the following claims.

## Claims

1. A medical image displaying method comprising:
displaying a medical image (641; 643) of an object captured in real time (S510), and a first reference image of the object registered with the medical image;
obtaining image information of a region adjacent to a first region (S520) within the medical image (641; 643), wherein the first region (711; is a distorted region of the medical image (641; 643) with respect to a shape of the object;
predicting image information of the first region (S530), based on the image information of the region (721) adjacent to the first region; and
reconstructing a region corresponding to the object within the first reference image (S540), based on the predicted image information of the first region, and displaying the reconstructed region.

2. The medical image displaying method of claim 1, wherein the obtaining of the image information of the region (721) adjacent to the first region comprises at least one of:
extracting a feature point of the object from the region (721) adjacent to the first region;
extracting a brightness value of the region (721) adjacent to the first region;
extracting a contour of the region (721) adjacent to the first region;
extracting variations in pixel values of the region (721) adjacent to the first region by using an image processing filter;
extracting histogram information of the region (721) adjacent to the first region;
extracting one or more differences between the pixel values of the region (721) adjacent to the first region; and
extracting shape information of the region (721) adjacent to the first region.

3. The medical image displaying method of claim 1, wherein the obtaining of the image information of the region (721) adjacent to the first region comprises:
determining, as the first region, a region having low image quality within the region corresponding to the object in the medical image; and
obtaining the image information of the region (721) adjacent to the first region.

4. The medical image displaying method of claim 1, wherein the predicting of the image information of the first region (902) comprises:
determining a shape of the region adjacent to the first region (902), based on the image information of the region adjacent to the first region (S531); and
predicting a shape of the first region (902) based on the determined shape of the region (901) adjacent to the first region, wherein the predicted shape of the first region (902) mirrors the determined shape of the region (901) adjacent to the first region with respect to an arbitrary axis (S532).

5. The medical image displaying method of claim 1, wherein the predicting of the image information of the first region comprises:
determining a shape of the region (904) adjacent to the first region (905), based on the image information of the region (904) adjacent to the first region (S531); and
predicting a shape of the first region (905) according to a predetermined pattern, when the determined shape of the region (904) adjacent to the first region corresponds to the predetermined pattern (S533).

6. The medical image displaying method of claim 1, wherein the reconstructing of the region corresponding to the object (S540) and displaying the reconstructed region within the first reference image comprises:
reconstructing the region corresponding to the object within the first reference image, based on the predicted image information of the first region; and
displaying the reconstructed region within the first reference image such that the reconstructed region is distinguished from an original region in the first reference image.

7. The medical image displaying method of claim 1, further comprising reconstructing the first region within the medical image and displaying the reconstructed first region (S540) such that the reconstructed first region overlaps the medical image.

8. The medical image displaying method of claim 1, wherein, when a type of the first reference image is different from a type of the medical image, the reconstructing of the region corresponding to the object and displaying the reconstructed region within the first reference image comprises:
reconstructing the first region within the medical image (S540);
converting coordinate information of the reconstructed first region from a coordinate system of the medical image to a coordinate system of the first reference image; and
displaying the region corresponding to the object within the first reference image, based on the converted coordinate information of the reconstructed first region.

9. The medical image displaying method of claim 1, further comprising:
displaying the obtained image information of the region adjacent to the first region and the predicted image information of the first region;
correcting the predicted image information of the first region (722), based on a user input (1101); and
reconstructing the region corresponding to the object and displaying the reconstructed region within the first reference image, based on the corrected image information of the first region.

10. The medical image displaying method of claim 1, further comprising magnifying and displaying the region corresponding to the object within the medical image,
wherein the reconstructing of the region corresponding to the object and displaying the reconstructed region within the first reference image (S540), based on the predicted image information of the first region (720), comprises magnifying and displaying the reconstructed region within the first reference image.

11. A medical image displaying apparatus (100; 300) comprising:
a display (320; 420) configured to display a medical image of an object captured in real time, and a first reference image of the object registered with the medical image; and
a processor (310) configured to obtain image information of a region (721) adjacent to a first region within the medical image, predict image information of the first region, based on the image information of the region (721) adjacent to the first region, and reconstruct a region corresponding to the object within the first reference image, based on the predicted image information of the first region, wherein the first region is a distorted region of the medical image (641; 643) with respect to a shape of the object,
wherein the display (320; 420) is adapted to display the reconstructed region.

12. The medical image displaying apparatus (100; 300) of claim 11, wherein the processor (310) is adapted to determine a shape of the region (721) adjacent to the first region, based on the image information of the region (721) adjacent to the first region, and is adapted to predict a shape (722) of the first region based on the determined shape of the region (721) adjacent to the first region, wherein the predicted shape (722) of the first region mirrors the determined shape of the region (721) adjacent to the first region with respect to an arbitrary axis.

13. The medical image displaying apparatus of claim 11, wherein the processor (310) is adapted to determine a shape of the region (721) adjacent to the first region, based on the image information of the region (721) adjacent to the first region, and is adapted to predict a shape (722) of the first region according to a predetermined pattern, when the determined shape of the region (721) adjacent to the first region corresponds to the predetermined pattern.

14. The medical image displaying apparatus of claim 11, further comprising a user interface (430),
wherein
the display is adapted to display the obtained image information of the region (721) adjacent to the first region and the predicted image information of the first region,
the user interface is adapted to receive a user input (1101) for correcting the predicted image information of the first region, and
the processor is adapted to correct the predicted image information of the first region, based on the user input (1101), is adapted to reconstruct the region corresponding to the object within the first reference image, based on the corrected image information of the first region, and is adapted to control the display to display the reconstructed region.

15. A computer program comprising instructions which, when the program is executed by a computing device, cause the computing device to carry out the method of claim 11.

## Patentansprüche

1. Verfahren zur Anzeige medizinischer Bilder, wobei das Verfahren Folgendes umfasst:
Anzeigen eines in Echtzeit aufgenommenen medizinischen Bildes (641; 643) eines Objektes (S510), und eines mit dem medizinischen Bild registrierten ersten Referenzbildes;
Erhalten von Bildinformationen eines an einen ersten Bereich innerhalb des medizinischen Bildes (641; 643) angrenzenden Bereichs (S520), wobei es sich bei dem ersten Bereich (711) um einen verzerrten Bereich des medizinischen Bildes (641; 643) in Bezug auf eine Form des Objektes handelt;
Vorhersagen von Bildinformationen des ersten Bereichs (S530) basierend auf den Bildinformationen des an den ersten Bereich angrenzenden Bereichs (721); und
Rekonstruieren eines dem Objekt entsprechenden Bereichs innerhalb des ersten Referenzbildes (S540) basierend auf den vorhergesagten Bildinformationen des ersten Bereichs, und Anzeigen des rekonstruierten Bereichs.

2. Verfahren zur Anzeige medizinischer Bilder nach Anspruch 1, wobei das Erhalten der Bildinformationen des an den ersten Bereich angrenzenden Bereichs (721) wenigstens eines der Folgenden umfasst:
Extrahieren eines Merkmalspunktes des Objektes aus dem an den ersten Bereich angrenzenden Bereich (721);
Extrahieren eines Helligkeitswertes des an den ersten Bereich angrenzenden Bereichs (721);
Extrahieren eines Umrisses des an den ersten Bereich angrenzenden Bereichs (721);
Extrahieren von Abweichungen der Pixelwerte des an den ersten Bereich angrenzenden Bereichs (721) unter Verwendung eines Bildverarbeitungsfilters;
Extrahieren von Histogramminformationen des an den ersten Bereich angrenzenden Bereichs (721);
Extrahieren von einem oder mehreren Unterschieden zwischen den Pixelwerten des an den ersten Bereich angrenzenden Bereichs (721); und
Extrahieren von Forminformationen des an den ersten Bereich angrenzenden Bereichs (721).

3. Verfahren zur Anzeige medizinischer Bilder nach Anspruch 1, wobei das Erhalten der Bildinformationen des an den ersten Bereich angrenzenden Bereichs (721) Folgendes umfasst:
Bestimmen eines Bereichs mit einer niedrigen Bildqualität innerhalb des dem Objekt entsprechenden Bereichs in dem medizinischen Bild als den ersten Bereich; und
Erhalten der Bildinformationen des an den ersten Bereich angrenzenden Bereichs (721).

4. Verfahren zur Anzeige medizinischer Bilder nach Anspruch 1, wobei das Vorhersagen der Bildinformationen des ersten Bereichs (902) Folgendes umfasst:
Bestimmen einer Form des an den ersten Bereich (902) angrenzenden Bereichs basierend auf den Bildinformationen des an den ersten Bereich angrenzenden Bereichs (S531); und
Vorhersagen einer Form des ersten Bereichs (902) basierend auf der bestimmten Form des an den ersten Bereich angrenzenden Bereichs (901), wobei die vorhergesagte Form des ersten Bereichs (902) die bestimmte Form des an den ersten Bereich angrenzenden Bereichs (901) in Bezug auf eine beliebige Achse widerspiegelt (S532).

5. Verfahren zur Anzeige medizinischer Bilder nach Anspruch 1, wobei das Vorhersagen der Bildinformationen des ersten Bereichs Folgendes umfasst:
Bestimmen einer Form des an den ersten Bereich (905) angrenzenden Bereichs (904) basierend auf den Bildinformationen des an den ersten Bereich angrenzenden Bereichs (904) (S531); und
Vorhersagen einer Form des ersten Bereichs (905) gemäß einem vorbestimmten Muster, wenn die bestimmte Form des an den ersten Bereich angrenzenden Bereichs (904) dem vorbestimmten Muster entspricht (S533).

6. Verfahren zur Anzeige medizinischer Bilder nach Anspruch 1, wobei die Rekonstruktion des dem Objekt entsprechenden Bereichs (S540) und das Anzeigen des rekonstruierten Bereichs innerhalb des ersten Referenzbildes Folgendes umfassen:
Rekonstruieren des dem Objekt entsprechenden Bereichs innerhalb des ersten Referenzbildes basierend auf den vorhergesagten Bildinformationen des ersten Bereichs; und
Anzeigen des rekonstruierten Bereichs innerhalb des ersten Referenzbildes derart, dass der rekonstruierte Bereich sich von einem ursprünglichen Bereich in dem ersten Referenzbild unterscheidet.

7. Verfahren zur Anzeige medizinischer Bilder nach Anspruch 1, weiterhin umfassend ein Rekonstruieren des ersten Bereichs innerhalb des medizinischen Bildes und Anzeigen des rekonstruierten ersten Bereichs (S540) derart, dass der rekonstruierte erste Bereich mit dem medizinischen Bild überlappt.

8. Verfahren zur Anzeige medizinischer Bilder nach Anspruch 1, wobei, wenn eine Art des ersten Referenzbildes sich von einer Art des medizinischen Bildes unterscheidet, die Rekonstruktion des dem Objekt entsprechenden Bereichs und das Anzeigen des rekonstruierten Bereichs innerhalb des ersten Referenzbildes Folgendes umfassen:
Rekonstruieren des ersten Bereichs innerhalb des medizinischen Bildes (S540);
Umwandeln von Koordinateninformationen des rekonstruierten ersten Bereichs von einem Koordinatensystem des medizinischen Bildes zu einem Koordinatensystem des ersten Referenzbildes; und
Anzeigen des dem Objekt entsprechenden Bereichs innerhalb des ersten Referenzbildes basierend auf den umgewandelten Koordinateninformationen des rekonstruierten ersten Bereichs:

9. Verfahren zur Anzeige medizinischer Bilder nach Anspruch 1, das weiterhin Folgendes umfasst:
Anzeigen der erhaltenen Bildinformationen des an den ersten Bereich angrenzenden Bereichs und der vorhergesagten Bildinformationen des ersten Bereichs;
Korrigieren der vorhergesagten Bildinformationen des ersten Bereichs (722) basierend auf einer Benutzereingabe (1101); und
Rekonstruieren des dem Objekt entsprechenden Bereichs und Anzeigen des rekonstruierten Bereichs innerhalb des ersten Referenzbildes basierend auf den korrigierten Bildinformationen des ersten Bereichs.

10. Verfahren zur Anzeige medizinischer Bilder nach Anspruch 1, weiterhin umfassend ein Vergrößern und Anzeigen des dem Objekt entsprechenden Bereichs innerhalb des medizinischen Bildes,
wobei das Rekonstruieren des dem Objekt entsprechenden Bereichs und Anzeigen des rekonstruierten Bereichs innerhalb des ersten Referenzbildes (S540) basierend auf den vorhergesagten Bildinformationen des ersten Bereichs (720) ein Vergrößern und Anzeigen des rekonstruierten Bereichs innerhalb des ersten Referenzbildes umfasst.

11. Vorrichtung (100, 300) zur Anzeige medizinischer Bilder, die Folgendes umfasst:
eine Anzeige (320; 420), die konfiguriert ist zum Anzeigen eines in Echtzeit aufgenommenen medizinischen Bildes eines Objektes, und eines mit dem medizinischen Bild registrierten ersten Referenzbildes; und
einen Prozessor (310), der zu Folgendem konfiguriert ist: Erhalten von Bildinformationen eines an einen ersten Bereich innerhalb des medizinischen Bildes angrenzenden Bereichs (721),
Vorhersagen von Bildinformationen des ersten Bereichs basierend auf den Bildinformationen des an den ersten Bereich angrenzenden Bereichs (721), und Rekonstruieren eines dem Objekt entsprechenden Bereichs innerhalb des ersten Referenzbildes basierend auf den vorhergesagten Bildinformationen des ersten Bereichs, wobei es sich bei dem ersten Bereich um einen verzerrten Bereich des medizinischen Bildes (641; 643) in Bezug auf eine Form des Objektes handelt,
wobei die Anzeige (320; 420) dazu angepasst ist, den rekonstruierten Bereich anzuzeigen.

12. Vorrichtung (100, 300) zur Anzeige medizinischer Bilder nach Anspruch 11, wobei der Prozessor (310) dazu angepasst ist, eine Form des an den ersten Bereich angrenzenden Bereichs (721) zu bestimmen basierend auf den Bildinformationen des an den ersten Bereich angrenzenden Bereichs (721), und
dazu angepasst ist, eine Form (722) des ersten Bereichs basierend auf der bestimmten Form des an den ersten Bereich angrenzenden Bereichs (721) zu bestimmen, wobei die vorhergesagte Form (722) des ersten Bereichs die bestimmte Form des an den ersten Bereich angrenzenden Bereichs (721) in Bezug auf eine beliebige Achse widerspiegelt.

13. Vorrichtung zur Anzeige medizinischer Bilder nach Anspruch 11, wobei der Prozessor (310) dazu angepasst ist, eine Form des an den ersten Bereich angrenzenden Bereichs (721) zu bestimmen basierend auf den Bildinformationen des an den ersten Bereich angrenzenden Bereichs (721), und
dazu angepasst ist, eine Form (722) des ersten Bereichs gemäß einem vorbestimmten Muster vorherzusagen, wenn die bestimmte Form des an den ersten Bereich angrenzenden Bereichs (721) dem vorbestimmten Muster entspricht.

14. Vorrichtung zur Anzeige medizinischer Bilder nach Anspruch 11, weiterhin umfassend eine Benutzerschnittstelle (430), wobei
die Anzeige angepasst ist zum Anzeigen der erhaltenen Bildinformationen des an den ersten Bereich angrenzenden Bereichs (721) und der vorhergesagten Bildinformationen des ersten Bereichs,
die Benutzerschnittstelle angepasst ist zum Empfangen einer Benutzereingabe (1101) zur Korrektur der vorhergesagten Bildinformationen des ersten Bereichs, und
der Prozessor angepasst ist zum Korrigieren der vorhergesagten Bildinformationen des ersten Bereichs basierend auf der Benutzereingabe (1101), angepasst ist zum Rekonstruieren des dem Objekt entsprechenden Bereichs in dem ersten Referenzbild basierend auf den korrigierten Bildinformationen des ersten Bereichs, und angepasst ist zum Steuern der Anzeige derart, dass sie den rekonstruierten Bereich anzeigt.

15. Computerprogramm, das Anweisungen umfasst, die, wenn das Programm von einer Computervorrichtung ausgeführt wird, die Computervorrichtung veranlassen, das Verfahren nach Anspruch 11 auszuführen.

## Revendications

1. Procédé d'affichage d'image médicale comprenant :
l'affichage d'une image médicale (641 ; 643) d'un objet prise en temps réel (S510), et d'une première image de référence de l'objet recalée sur l'image médicale,
l'obtention d'informations d'image d'une zone adjacente à une première zone (S520) au sein de l'image médicale (641 ; 643), ladite première zone (711) étant une zone déformée de l'image médicale (641 ; 643) par rapport à une forme de l'objet,
la prédiction d'informations d'image de la première zone (S530), compte tenu des informations d'image de la zone (721) adjacente à la première zone, et
la reconstruction d'une zone correspondant à l'objet au sein de la première image de référence (S540), compte tenu des informations d'image prédites de la première zone, et l'affichage de la zone reconstruite.

2. Procédé d'affichage d'image médicale selon la revendication 1, dans lequel l'obtention des informations d'image de la zone (721) adjacente à la première zone comprend au moins l'une des extractions suivantes :
l'extraction d'un point caractéristique de l'objet à partir de la zone (721) adjacente à la première zone,
l'extraction d'une valeur de luminosité de la zone (721) adjacente à la première zone,
l'extraction d'un contour de la zone (721) adjacente à la première zone,
l'extraction de variations concernant des valeurs de pixels de la zone (721) adjacente à la première zone au moyen d'un filtre de traitement d'image,
l'extraction d'informations d'histogramme de la zone (721) adjacente à la première zone,
l'extraction d'une ou plusieurs différences entre les valeurs de pixels de la zone (721) adjacente à la première zone, et
l'extraction d'informations de forme de la zone (721) adjacente à la première zone.

3. Procédé d'affichage d'image médicale selon la revendication 1, dans lequel l'obtention des informations d'image de la zone (721) adjacente à la première zone comprend :
la détermination, comme première zone, d'une zone ayant une moindre qualité d'image au sein de la zone correspondant à l'objet dans l'image médicale, et
l'obtention des informations d'image de la zone (721) adjacente à la première zone.

4. Procédé d'affichage d'image médicale selon la revendication 1, dans lequel la prédiction des informations d'image de la première zone (902) comprend :
la détermination d'une forme de la zone adjacente à la première zone (902), compte tenu des informations d'image de la zone adjacente à la première zone (S531), et
la prédiction d'une forme de la première zone (902) compte tenu de la forme déterminée de la zone (901) adjacente à la première zone, ladite forme prédite de la première zone (902) reproduisant en symétrie la forme déterminée de la zone (901) adjacente à la première zone par rapport à un axe arbitraire (S532).

5. Procédé d'affichage d'image médicale selon la revendication 1, dans lequel la prédiction des informations d'image de la première zone comprend :
la détermination d'une forme de la zone (904) adjacente à la première zone (905), compte tenu des informations d'image de la zone (904) adjacente à la première zone (S531), et
la prédiction d'une forme de la première zone (905) selon un motif prédéterminé, lorsque la forme déterminée de la zone (904) adjacente à la première zone correspond au motif prédéterminé (S533).

6. Procédé d'affichage d'image médicale selon la revendication 1, dans lequel la reconstruction de la zone correspondant à l'objet (S540) et l'affichage de la zone reconstruite au sein de la première image de référence comprennent :
la reconstruction de la zone correspondant à l'objet au sein de la première image de référence, compte tenu des informations d'image prédites de la première zone, et
l'affichage de la zone reconstruite au sein de la première image de référence de façon que la zone reconstruite se distingue d'une zone originale de la première image de référence.

7. Procédé d'affichage d'image médicale selon la revendication 1, comprenant en outre la reconstruction de la première zone au sein de l'image médicale et l'affichage de la première zone reconstruite (S540) de façon que la première zone reconstruite soit en chevauchement avec l'image médicale.

8. Procédé d'affichage d'image médicale selon la revendication 1, dans lequel, lorsque le type de la première image de référence est différent du type de l'image médicale, la reconstruction de la zone correspondant à l'objet et l'affichage de la zone reconstruite au sein de la première image de référence comprennent :
la reconstruction de la première zone au sein de l'image médicale (S540),
la conversion d'informations de coordonnées de la première zone reconstruite à partir d'un système de coordonnées de l'image médicale en un système de coordonnées de la première image de référence, et
l'affichage de la zone correspondant à l'objet au sein de la première image de référence, compte tenu des informations de coordonnées de la première zone reconstruite converties.

9. Procédé d'affichage d'image médicale selon la revendication 1, comprenant en outre :
l'affichage des informations d'image obtenues de la zone adjacente à la première zone et des informations d'image prédites de la première zone,
la correction des informations d'image prédites de la première zone (722), compte tenu d'une entrée utilisateur (1101), et
la reconstruction de la zone correspondant à l'objet et l'affichage de la zone reconstruite au sein de la première image de référence, compte tenu des informations d'image corrigées de la première zone.

10. Procédé d'affichage d'image médicale selon la revendication 1, comprenant en outre l'agrandissement et l'affichage de la zone correspondant à l'objet au sein de l'image médicale, ladite reconstruction de la zone correspondant à l'objet et l'affichage de la zone reconstruite au sein de la première image de référence (S540), compte tenu des informations d'image prédites de la première zone (720), comprenant l'agrandissement et l'affichage de la zone reconstruite au sein de la première image de référence.

11. Dispositif d'affichage d'image médicale (100 ; 300) comprenant :
un écran (320 ; 420) conçu pour afficher une image médicale d'un objet prise en temps réel, et une première image de référence de l'objet recalée sur l'image médicale, et
un processeur (310) conçu pour obtenir des informations d'image d'une zone (721) adjacente à une première zone au sein de l'image médicale, prédire des informations d'image de la première zone, compte tenu des informations d'image de la zone (721) adjacente à la première zone, et
reconstruire une zone correspondant à l'objet au sein de la première image de référence, compte tenu des informations d'image prédites de la première zone, ladite première zone étant une zone déformée de l'image médicale (641 ; 643) par rapport à une forme de l'objet,
ledit écran (320 ; 420) étant apte à afficher la zone reconstruite.

12. Dispositif d'affichage d'image médicale (100 ; 300) selon la revendication 11, dans lequel le processeur (310) est apte à déterminer une forme de la zone (721) adjacente à la première zone, compte tenu des informations d'image de la zone (721) adjacente à la première zone, et
est apte à prédire une forme (722) de la première zone compte tenu de la forme déterminée de la zone (721) adjacente à la première zone, ladite forme prédite (722) de la première zone reproduisant en symétrie la forme déterminée de la zone (721) adjacente à la première zone par rapport à un axe arbitraire.

13. Dispositif d'affichage d'image médicale selon la revendication 11, dans lequel le processeur (310) est apte à déterminer une forme de la zone (721) adjacente à la première zone, compte tenu des informations d'image de la zone (721) adjacente à la première zone, et
est apte à prédire une forme (722) de la première zone selon un motif prédéterminé, lorsque la forme déterminée de la zone (721) adjacente à la première zone correspond au motif prédéterminé.

14. Dispositif d'affichage d'image médicale selon la revendication 11, comprenant en outre une interface utilisateur (430) et dans lequel
l'écran est apte à afficher les informations d'image obtenues de la zone (721) adjacente à la première zone et les informations d'image prédites de la première zone,
l'interface utilisateur est apte à recevoir une entrée utilisateur (1101) pour corriger les informations d'image prédites de la première zone, et
le processeur est apte à corriger les informations d'image prédites de la première zone, compte tenu de l'entrée utilisateur (1101), est apte à reconstruire la zone correspondant à l'objet au sein de la première image de référence, compte tenu des informations d'image corrigées de la première zone, et est apte à commander l'écran de façon à afficher la zone reconstruite.

15. Programme informatique comprenant des instructions qui, lorsque le programme est exécuté par un dispositif informatique, amènent le dispositif informatique à mettre en œuvre le procédé selon la revendication 11.
